# EUROPÄISCHE PATENTANMELDUNG

(11) **EP 4 710 898 A1**
(43) Veröffentlichungstag der Anmeldung: **18.03.2026**
(21) Anmeldenummer: 25201803.1
(22) Anmeldetag: 12.09.2025
(51) Int. Cl.: A61F 5/01, A41B 11/02, A43B 7/14, A61F 5/14

(54) **KIT ZUM AUSGLEICH VON FEHLSTELLUNG UND/ODER FEHLBELASTUNGEN**

(30) Priorität: 13.09.2024 DE 102024126488; 12.12.2024 EP 24219493
(71) Anmelder: Kumo Holding GmbH, 45257 Essen (DE)
(72) Erfinder: KUNZE, Patrick, 45529 Hattingen (DE)
(74) Vertreter: Von Rohr Patentanwälte Partnerschaft mbB

(57) **Zusammenfassung**

Es wird ein Kit zum Ausgleich einer Fehlstellung und/oder Fehlbelastung des Bewegungsapparats einer Person vorgeschlagen. Das Kit weist einen Fußüberzieher zur Anlage am Fuß der Person auf. Der Fußüberzieher bedeckt wenigstens einen Abschnitt des Fußes und/oder umgibt den Fuß zumindest abschnittsweise vollumfänglich. Das Kit weist des Weiteren ein Stützelement zum Stützen und/oder Anheben eines Fußabschnittsauf.

## Beschreibung

Die Erfindung betrifft ein Kit zum Ausgleich von Fehlstellung und/oder Fehlbelastungen des Bewegungsapparats einer Person gemäß Anspruch 1.

Zur Linderung von Beschwerden bei Fehlstellung und/oder Fehlbelastungen des Bewegungsapparats einer Person sind Einlegesohlen bekannt. Durch entsprechende Ausbildung der Einlegesohle, insbesondere durch speziell angeordnet Erhöhungen, Vertiefungen und/oder Konturen, kann eine Fehlstellung und/oder Fehlbelastung des Bewegungsapparats der Person zielgerichtet und individuell ausgeglichen werden.

Einlegesohlen werden in der Regel für die Person maßgefertigt und an einen Schuh unmittelbar angepasst, was aufwendig sowie zeit- und kostenintensiv ist. Schuhe von verschiedenen Herstellern und/oder verschiedene Schuhmodelle weisen bei identischen Schuhgrößen häufig unterschiedlich große Sohlen und/oder Sohlen unterschiedlicher Form auf, wodurch die Einlegesohlen zwar in einem Paar Schuhe optimal einliegen, in einem anderen Paar der Person jedoch beim Tragen der Schuhe relativ zum Schuh verrutschen können. Ein Ausgleich der Fehlstellung und/oder Fehlbelastung ist dann nicht mehr sichergestellt. Alternativ oder zusätzlich kann eine verrutsche Einlegesohle als unangenehm beim Laufen empfunden werden und zu anderweitigen Fehstellungen und/oder Fehlbelastungen des Bewegungsapparats der Person führen.

Darüber hinaus muss beim Auftreten von weiteren Fehlstellungen und/oder Fehlbelastungen des Bewegungsapparats in der Regel eine neue Einlegesohle maßgefertigt werden, was zu hohen Kosten und langen Wartezeiten führt.

Aus dem Stand der Technik sind auch Einlegesohlen und Socken bekannt, bei denen Stützelemente an der Einlegesohle bzw. Socke befestigt werden.

US 2017/0295884 A1 offenbart ein modulares Fußbett-System in der Art eines Pantoffels, bei dem Aufnahmen für orthopädische Stützelemente vorgesehen sind. Die Aufnahmen können mithilfe von lösbaren Verbindungsmitteln, wie Klettstreifen oder Klebestreifen, an unterschiedlichen Positionen fixiert und ausgetauscht werden. Die Stützelemente werden über die Öffnungen in die Aufnahmen eingeschoben.

US 1,797,143 A offenbart eine Fußbett-Einlage aus weichem Gummimaterial mit integrierten Taschen zur Aufnahme individuell anpassbarer Poster für den Fersenbereich, das Fußgewölbe und den Ballen.

US 2006/0000120 A1 offenbart eine orthopädische Einlegesohle aus elastischem Material mit profilierten Elementen auf der Oberseite zur Aktivierung von Gelenkrezeptoren und zur Führung des Fußes entlang der physiologischen Abrollachse. Auf der Unterseite weist die Sohle mehrere passgenaue Ausnehmungen auf, in die formkorrespondierende, steifere Stützelemente befestigt werden können.

KR 10-2016-0047901 offenbart eine Socke, die mit mehreren Stützelementen ausgestattet ist, um das Fußgewölbe dauerhaft zu unterstützen. Die Stützelemente sind an der Innenseite der Sohle der Socke angebracht und bestehen aus elastischem Material.

Es ist daher Aufgabe der vorliegenden Erfindung, die Nachteile des bekannten Standes der Technik zu überwinden bzw. ein verbessertes Kit zum Ausgleich von Fehlstellung und/oder Fehlbelastungen des Bewegungsapparats einer Person bereitzustellen.

Die obige Aufgabe wird durch ein Kit zum Ausgleich von Fehlstellung und/oder Fehlbelastungen des Bewegungsapparats einer Person gemäß Anspruch 1 gelöst. Vorteilhafte Weiterbildungen sind Gegenstand der Unteransprüche.

Das erfindungsgemäße Kit ist zum Ausgleich einer Fehlstellung und/oder Fehlbelastung des Bewegungsapparats einer Person ausgebildet.

Das Kit weist einen Fußüberzieher für einen Fuß der Person auf, insbesondere zur Anlage an dem Fuß. In einem Tragezustand bedeckt der Fußüberzieher wenigstens einen Abschnitt des Fußes. Alternativ oder zusätzlich umgibt bzw. umschließt der Fußüberzieher im Tragezustand den Fuß zumindest abschnittsweise vollumfänglich.

Unter dem Begriff "Tragezustand" ist vorzugsweise der Zustand des Kits zu verstehen, in dem die Person den Fußüberzieher trägt bzw. der Fußüberzieher ordnungsgemäß am Fuß anliegt oder diesen wenigstens abschnittsweise ordnungsgemäß umgibt, um eine Fehlstellung und/oder Fehbelastung des Bewegungsapparats der Person auszugleichen.

Unter dem Begriff "ausgleichen" ist vorliegend ein wenigstens teilweises Ausgleichen einer Fehlstellung und/oder Fehlbelastung des Bewegungsapparats einer Person zu verstehen.

Das Kit weist des Weiteren vorzugsweise ein Stützelement zum Stützen und/oder Anheben eines Fußabschnitts der Person auf. Durch das Stützen und/oder Anheben des entsprechenden Fußabschnitts kann eine Fehlstellung und/oder Fehlbelastung des Bewegungsapparats der Person ausgeglichen werden.

Das Stützelement ist vorzugsweise an wenigstens zwei verschiedenen vorgegeben Bereichen des Fußüberziehers lösbar befestigbar, um jeweils unterschiedliche Fehlstellungen und/oder Fehlbelastungen des Bewegungsapparats auszugleichen. In Abhängigkeit davon, an welchem Bereich des Fußüberziehers das Stützelement befestigt oder angebracht ist bzw. wird, können unterschiedliche Fehlstellungen und/oder Fehlbelastungen des Bewegungsapparats der Person ausgeglichen werden.

So kann beispielsweise bei einer Anordnung des Stützelements an einem ersten Bereich eine erste Fehlstellung und/oder Fehlbelastung des Bewegungsapparats ausgeglichen werden. Bei einer Anordnung des Stützelements an einem zweiten Bereich kann eine zweite Fehlstellung und/oder Fehlbelastung des Bewegungsapparats der Person ausgeglichen werden. Auf diese Weise können mit einem erfindungsgemäßen Kit unterschiedliche Fehlstellungen und/oder Fehlbelastungen des Bewegungsapparats ausgeglichen werden. Es ist dann nicht notwendig, für jede Fehlstellung und/oder Fehlbelastung eine separate Einlegesohle und/oder ein separates Kit vorzusehen.

Zudem ist das Kit unabhängig von einem Paar Schuhe verwendbar. Durch Verwendung des Kits können die Schuhe beliebig gewechselt werden, was bei bekannten Einlegesohlen aufgrund der Unterschiedlichen Form und Größe den Sohlen der Schuhe nicht immer möglich ist.

Das Kit bietet darüber hinaus den weiteren Vorteil, dass Fehlstellungen und/oder Fehlbelastungen auch dann ausgeglichen werden können, wenn die Person keine Schuhe trägt. Die Unabhängigkeit des Ausgleichs der Fehlstellungen und/oder Fehlbelastungen von Schuhen ist dahingehend besonders vorteilhaft, da nicht an allen Orten Schuhe getragen werden können. So ist es in vielen Kulturkreisen nicht akzeptiert, im Inneren von Wohngebäuden oder Gebetstätten Schuhe zu tragen. Dies ist für betroffene Personen häufig mit Schwierigkeiten verbunden, da ihre Fehlstellungen und/oder Fehlbelastungen während eines Aufenthalts in entsprechenden Räumlichkeiten nicht ausgeglichen werden können. Hier schafft das erfindungsgemäße Kit entsprechend Abhilfe.

Gemäß einer weiter bevorzugten Ausführungsform weist der Fußüberzieher wenigstens zwei oder drei, vorzugsweise wenigstens vier, weiter vorzugsweise wenigstens fünf, weiter vorzugsweise wenigstens sechs, weiter vorzugsweise genau sieben, unterschiedliche vorgegebene Bereiche auf, wodurch eine Vielzahl von Fehlstellungen und/oder Fehlbelastungen des Bewegungsapparats in besonders guter Weise ausgeglichen werden können.

Die vorgegebenen Positionen auf dem Fußüberzieher können derart ausgebildet sein, dass die unterschiedlichen Bereiche voneinander unterscheidbar sind. Insbesondere können die vorgegebenen Bereiche markiert sein, beispielsweise farblich. So kann jeder Bereich mittels einer anderen Farbe markiert sein. Es ist alternativ oder zusätzlich möglich, dass die Konturen bzw. Formen der Bereiche unterschiedlich ausgebildet sind.

Das Stützelement kann zumindest teilweise elastisch verformbar sein, um sich der Form des Fußes anzupassen und/oder um der Bildung von Druckstellen entgegenzuwirken oder die Bildung von Druckstellen zu verhindern.

Das Stützelement kann aus einem Kunststoffmaterial hergestellt sein, wobei zusätzlich oder alternativ auch andere Materialien verwendet werden können.

Gemäß einer besonders bevorzugten Ausführungsform weist das Kit wenigstens zwei, weiter vorzugsweise wenigstens drei, insbesondere genau sieben, Stützelemente auf, vorzugsweise wobei im Tragezustand ein Stützelement an einem vorgegebenen Bereich oder eine Mehrzahl an Stützelementen an verschiedenen vorgegebenen Bereichen des Fußüberziehers lösbar befestigt ist bzw. sind.

Besonders bevorzugt sind die Stützelemente unterschiedlich ausgebildet. Auf diese Weise kann für den Ausgleich unterschiedlicher Fehlstellungen und/oder Fehlbelastungen ein an den jeweiligen Anwendungsfall angepasstes Stützelement verwendet werden.

Die Bereiche auf dem Fußüberzieher sind vorzugsweise unterschiedlich ausgebildet, um die Zuordnung zu einem Stützelement zu erleichtern. So können die einzelnen Bereiche beispielsweise unterschiedliche Formen und/oder Konturen aufweisen. Zusätzlich oder alternativ können die verschiedenen Bereiche auch unterschiedlich farblich oder durch eine Beschriftung markiert sein. Insbesondere können die verschiedenen Bereiche farblich umrandet sein und/oder durch eine farbliche Naht hervorgehoben sein.

Zusätzlich oder alternativ sind die Stützelemente unterschiedlich markiert, beispielsweise durch unterschiedliche Farben und/oder jeweils eine Beschriftung. Ganz besonders bevorzugt korrespondiert die Markierung der Stützelemente zu der Markierung der Bereiche auf dem Fußüberzieher.

Das Stützelement weist vorzugsweise eine dem Fußüberzieher zugewandte Anlagefläche auf. Der Fußüberzieher weist vorzugsweise eine dem Stützelement zugeordnete und zugewandte Anlagegegenfläche weist, wobei die Anlagefläche und die Anlagegegenfläche zumindest im Wesentlichen gleich, insbesondere komplementär zueinander, ausgebildet sind. Auf diese Weise kann die Platzierung bzw. Anordnung des Stützelements relativ zum Bereich des Fußüberziehers bzw. relativ zum Fußüberzieher in besonders einfacher und intuitiver Weise erfolgen.

Die Konturen des jeweiligen Bereichs und des dem Bereich zugeordneten Stützelements können somit komplementär zueinander und/oder identische ausgebildet sein.

Die Größe und/oder Form des vorgegebenen Bereichs kann der Anlagefläche und/oder der Anlagegegenfläche entsprechen, wodurch die vollständige Fläche des vorgegebenen Bereichs zur Ausbildung einer besonders festen und/oder stabilen Verbindung genutzt werden kann.

Bevorzugt ist die Verbindung zwischen dem Fußüberzieher und dem Stützelement mittels einer Klettverschlussverbindung realisiert, wobei auch andere wieder lösbare Verbindungsarten möglich sind.

Gemäß einer besonders bevorzugten Ausführungsform entspricht die Anzahl der Bereiche der Anzahl der Stützelemente. Insbesondere kann für jeden vorgegebenen Bereich ein speziell ausgebildetes, insbesondere auf den jeweiligen Bereich abgestimmtes, Stützelement vorgesehen sein. Alternativ oder zusätzlich kann jedem vorgegebenen Bereich genau ein speziell ausgebildetes Stützelement oder jedem Stützelement genau ein vorgegebener Bereich zugeordnet sein. Auf diese Weise kann jedes Stützelement und/oder jeder Bereich zum Ausgleich einer oder mehrerer Fehlstellungen und/oder Fehlbelastungen in besonderem Maße angepasst sein. Somit kann die jeweilige Fehlstellung und/oder Fehlbelastung in besonders wirkungsvoller Weise ausgeglichen werden.

Zwei der vorgegebenen Bereiche können den Zehen der Person zugeordnet sein. So kann insbesondere ein Bereich dem großen Zeh bzw. Hallux und/oder ein Bereich den Digitus II bis V zugeordnet sein. Entsprechend kann ein Stützelement dem großen Zeh bzw. Hallux und/oder ein Stützelement den Digitus II bis V zugeordnet sein. Mit dem Kit können somit Fehlstellungen und/oder Fehlbelastungen durch Krallenzehen und/oder Wadenkrämpfe ausgeglichen werden.

Drei Bereiche können dem Mittelfuß der Person zugeordnet sein und/oder sich ausgehend von den Zehen zur Ferse erstrecken. Ein Bereich kann der Innenseite, ein Bereich der Außenseite und/oder ein Bereich dem Innenbereich des Mittelfußes zugeordnet sein.

Entsprechend können drei Stützelemente dem Mittelfuß der Person zugeordnet sein und/oder sich im Tragezustand ausgehend von den Zehen bis zur Ferse erstrecken. Ein Stützelement kann der Innenseite, einem Bereich der Außenseite und/oder einem Bereich dem Innenbereich des Mittelfußes zugeordnet sein.

Mit dem Kit können somit Fehlstellungen und/oder Fehlbelastungen bei Knieschmerzen, des Vorfußes und/oder wegen einer Hohlfußstellung ausgeglichen werden.

Zwei Bereiche können der Ferse der Person zugeordnet sein. Insbesondere kann ein Bereich der Innenseite der Ferse und ein Bereich der Außenseite der Ferse zugeordnet sein. Entsprechend können zwei Stützelemente der Ferse der Person zugeordnet sein, wobei ein Stützelement der Innenseite der Ferse bzw. der inneren Hälfte der Ferse und ein Stützelement der Außenseite der Ferse bzw. der äußeren Hälfte der Ferse zugeordnet sein kann. Auf diese Weise können Fehlstellungen und/oder Fehlbelastungen der Achillessehne und/oder aufgrund eines Fersensporns ausgeglichen werden.

Die dem Mittelfuß zugeordneten Bereiche und/oder Stützelemente können in distaler Richtung eine größere Erstreckung aufweisen als die den Zehen zugeordneten Bereiche und/oder Stützelemente und/oder die der Ferse zugeordneten Bereiche und/oder Stützelemente.

Alternativ oder zusätzlich können die der Ferse zugeordneten Bereiche und/oder Stützelemente eine größere Erstreckung in distaler Richtung aufweisen als die den Zehen zugeordneten Bereiche und/oder Stützelemente.

Der dem Innenbereich des Mittelfußes zugeordnete Bereich und/oder das dem **In**nenbereich des Mittelfußes zugeordnete Stützelement kann zumindest im Wesentlichen V-förmig, tropfenförmig und/oder drachendreieckförmig ausgebildet sein.

Aufgrund der entsprechenden Ausbildung der Bereiche und/oder der Stützelemente kann ein Ausgleich spezifischer Fehlstellungen und/oder Fehlbelastungen besonders wirkungsvoll erfolgen.

Gemäß einer besonders bevorzugten Ausführungsform kann jeder vorgegebene Bereich jeweils eine Aufnahmetasche zur Aufnahme eines Stützelements aufweisen oder durch eine Aufnahmetasche zur Aufnahme eines Stützelements gebildet sein, wobei das Stützelement zur Befestigung an dem Fußüberzieher - insbesondere über eine schlitzartige Öffnung - in die Aufnahmetasche einführbar ist. Auf diese Weise kann eine besonders feste Fixierung des Stützelements relativ zum Fußüberzieher erreicht werden.

Besonders bevorzugt kann die Aufnahmetasche eine vorzugsweise schlitzartige Öffnung aufweisen und derart dehnbar sein, dass das Stützelement in einem Öffnungszustand der Öffnung in die Aufnahmetasche einführbar ist. In einem Schließzustand der Öffnung ist das Stützelement vorzugsweise in der Aufnahmetasche fixiert. Durch die Dehnbarkeit der Aufnahmetasche kann somit zum einen eine einfache Einführung und Fixierung des Stützelement erzielt und zum anderen die Bildung von Druckstellen verhindert werden, da keine weiteren Komponenten zum Verschließen der Öffnung, wie beispielsweise Klettverschlusselemente und/oder Knöpfe notwendig sind.

Die Dehnbarkeit der Aufnahmetasche kann durch den Einsatz eines reversibel dehnbaren Materials, insbesondere mit hoher Elastizität, erreicht werden, beispielsweise durch den Einsatz Polychloropren (Neopren), von Elastan oder eines Elastan enthaltenden Materials.

Zur Entnahme des Stützelements aus der Aufnahmetasche kann das Stützelement in besonders einfacher Weise über die Öffnung aus der Aufnahmetasche entnommen werden.

Die Aufnahmetasche kann auf den Fußüberzieher, insbesondere einen Sohlenbereich des Fußüberziehers, aufgebracht, vorzugsweise aufgenäht sein. Insbesondere ist es möglich, dass ein Sohlenelement, welches die Aufnahmetaschen aufweist, auf dem Fußüberzieher, insbesondere einem Sohlenbereich des Fußüberziehers, aufgebracht, vorzugsweise aufgenäht ist. Auf diese Weise kann der Fußüberzieher und damit das Kit in besonders schneller und einfacher Weise hergestellt werden. Alternativ ist es auch möglich, dass die Aufnahmetaschen auf den Sohlenbereich des Fußüberziehers aufgebracht, vorzugsweise aufgenäht sind.

Um ein Ausrutschen der Person beim Tragen des Fußüberziehers ohne Schuhe zu verhindern, weist der Fußüberzieher vorzugsweise eine der Fußsohle abgewandte Bodenkontaktfläche auf, wobei der Fußüberzieher im Bereich der Bodenkontaktfläche eine Anti-Rutsch-Schicht aufweist. Alternativ oder zusätzlich kann das Stützelement im Tragezustand eine der Fußsohle abgewandte Bodenfläche aufweisen, wobei die Bodenfläche als Anti-Rutsch-Schicht ausgebildet ist. Die Bodenkontaktfläche und/oder der Fußüberzieher kann zumindest abschnittsweise aus einem Softshell-material oder einem anderen Textil hergestellt sein.

Bevorzugt ist der Fußüberzieher nach Art einer Socke oder sockenartig ausgebildet, wodurch eine besonders hohe Akzeptanz bei der tragenden Person erreicht werden kann.

Gemäß einer besonders vorteilhaften Ausführungsform kann das Kit ein vom Fußüberzieher separates Aufnahmeelement zur Aufnahme des Stützelements aufweisen. Das Aufnahmeelement ist vorzugsweise nach Art, insbesondere in Form, einer Schuhsohle, insbesondere Schuheinlegesohle, ausgebildet. Auf diese Weise können die Stützelemente derart an dem Aufnahmeelement angeordnet werden, wie sie auch am Fußüberzieher angeordnet bzw. angebracht werden. Die Person kann dann in besonders einfacher und intuitiver Weise erkennen, welches Stützelement welchem vorgegebenen Bereich des Fußüberziehers zuzuordnen ist. Das Aufnahmeelement kann alternativ auch als Kiste, Halterung oder dergleichen ausgebildet sein.

Die vorgenannten Aspekte und Merkmale der vorliegenden Erfindung sowie die sich aus der weiteren Beschreibung ergebenden Aspekte und Merkmale der vorliegenden Erfindung können unabhängig voneinander, aber auch in beliebiger Kombination realisiert werden.

Zusätzliche Vorteile, Merkmale, Eigenschaften und Aspekte der vorliegenden Erfindung ergeben sich aus den Ansprüchen und der folgenden Beschreibung und einer bevorzugten Ausführungsform anhand der Zeichnung. Es zeigt:
- Fig. 1: ein vorschlagsgemäßes Kit mit einem Fußüberzieher und einem Stützelement in einer perspektivischen Ansicht,
- Fig. 2: den Fußüberzieher mit einem separaten Stützelement gemäß einer Ausführungsform in einer Ansicht von unten,
- Fig. 3: den Fußüberziehers mit einem separaten Stützelement gemäß einer weiteren Ausführungsform in einer Ansicht von unten,
- Fig. 4: den Fußüberzieher aus Fig. 3 mit dem verbundenen Stützelement in einer Ansicht von unten, und
- Fig. 5: das vorschlagsgemäße Kit mit einem Fußüberzieher und sieben Stützelementen.

In den Figuren werden für gleiche oder ähnliche Teile dieselben Bezugszeichen verwendet, wobei entsprechende oder vergleichbare Vorteile erreicht werden, auch wenn eine wiederholte Beschreibung aus Vereinfachungsgründen weggelassen ist.

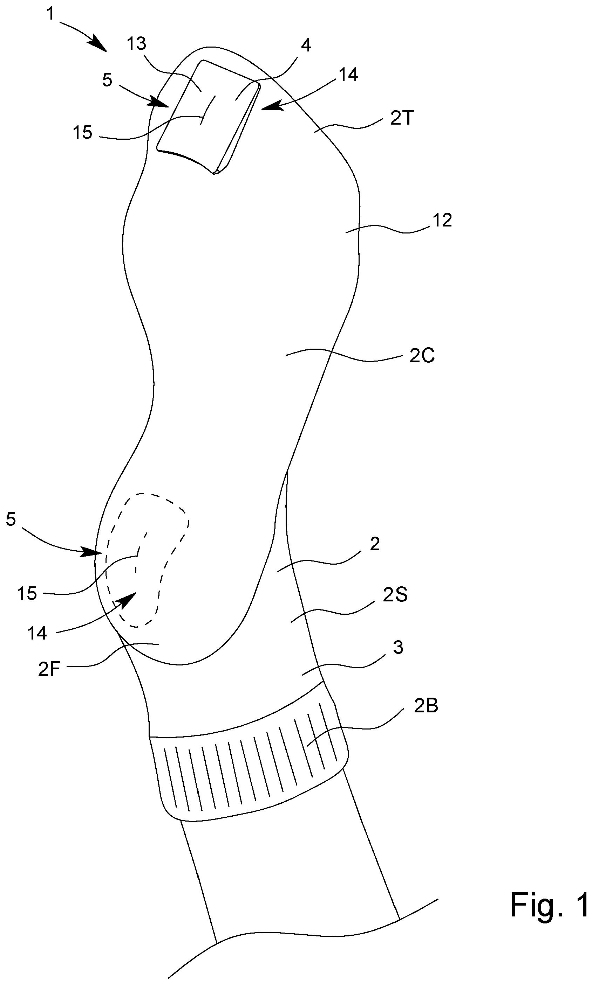

Fig. 1 zeigt ein Kit 1 zum Ausgleich einer Fehlstellung und/oder Fehlbelastung des Bewegungsapparats einer Person.

Das Kit 1 weist einen Fußüberzieher 2 für einen Fuß der Person auf. In Fig. 1 ist das Kit 1 bzw. der Fußüberzieher 2 in einem Tragezustand gezeigt, in dem die Person das Kit 1 bzw. den Fußüberzieher 2 am Fuß trägt. Es ist zwar nur ein Fußüberzieher 2 gezeigt. Das Kit 1 kann aber auch einen weiteren Fußüberzieher 2 aufweisen. Insbesondere kann das Kit 1 mit einem ersten bzw. rechten Fußüberzieher 2 für den rechten Fuß und einem zweiten bzw. linken Fußüberzieher für den linken Fuß ausgestattet sein. Im Folgenden wird lediglich ein Fußüberzieher 2 beschrieben. Einzelne, mehrere oder alle Eigenschaften, Merkmale oder Vorteile für den Fußüberzieher 2 gelten vorzugsweise auch für den anderen Fußüberzieher 2, auch wenn nicht explizit erläutert.

Vorzugsweise bedeckt der Fußüberzieher 2 im Tragezustand wenigstens einen Abschnitt des Fußes. Alternativ oder zusätzlich kann der Fußüberzieher 2 den Fuß im Tragezustand abschnittsweise vollumfänglich umgeben bzw. umschließen.

In der in den Figuren gezeigten und insoweit bevorzugten Ausführungsform ist der Fußüberzieher 2 nach Art einer Socke bzw. Strumpf und/oder sockenartig ausgebildet. Insbesondere kann der als Socke ausgebildete Fußüberzieher 2 über den Fuß gezogen und einer Socke entsprechend getragen werden. Insbesondere kann beim Tragen des Fußüberziehers 2 auch ein Schuh ordnungsgemäß getragen werden.

Es ist alternativ auch möglich, dass der Fußüberzieher 2 nach Art einer Schlauchbandage und/oder einer Knöchelbandage ausgebildet ist. Der Fuß kann dann nicht vollständig von dem Fußüberzieher 2 umgeben sein. So ist es beispielsweise möglich, dass die Zehen und/oder die Ferse nicht oder nicht vollständig vom Fußüberzieher 2 bedeckt sind.

Die folgende Beschreibung bezieht sich auf die bevorzugte Ausgestaltung des Fußüberziehers 2 als Socke, wobei die Ausführungen auch auf andere Ausgestaltungen des Fußüberziehers 2 übertragbar sind.

Der Fußüberzieher 2 ist vorzugsweise zumindest teilweise elastisch ausgebildet. Der Fußüberzieher 2 kann eine Kompression aufweisen bzw. bereitstellen oder als Kompressionssocke ausgebildet sein, um ein Verrutschen des Fußüberziehers 2 zu vermeiden.

Insbesondere kann der Fußüberzieher 2 aus einem Textil hergestellt sein. Bei dem Textil kann es sich um ein Gewebe, Gewirk und/oder Geflecht handeln. Es ist jedoch auch möglich, dass das Textil wenigstens ein Vlies und/oder ein Gelege aufweist. Das Textil bzw. der Fußüberzieher 2 kann aus Naturfaser, wie beispielsweise Baumwolle oder Bambus, und/oder aus Kunstfaser, wie beispielsweise Polyester, Elasthan oder dergleichen, gebildet sein.

Der Fußüberzieher 2 weist eine dem Fuß zugeordnete Innenseite und eine dem Fuß abgewandte Außenseite 3 auf. Vorzugsweise liegt die Innenseite unmittelbar gegen den Fuß an.

Der Fußüberzieher 2 weist vorzugsweise ein Bündchen 2B, einen Schaft 2S, eine Ferse 2F, einen Fußteil 2C und/oder eine optionale Fußspitze 2T auf.

Der Fußüberzieher 2 ist vorzugsweise zumindest bereichsweise, insbesondere auf einer Unterseite bzw. im Bereich der Fußsohle der tragenden Person, mehrlagig ausgebildet.

Vorzugsweise weist der Fußüberzieher 2 einen Grundkörper und eine Bodenkontaktschicht auf, insbesondere wobei die Bodenkontaktschicht mit dem Grundkörper kraft- und/oder stoffschlüssig verbunden, insbesondere vernäht und/oder verklebt, ist.

Die Bodenkontaktschicht bildet vorzugsweise eine Unterseite bzw. Sohle des Fußüberziehers 2 und/oder erstreckt sich von der Ferse 2F bis zur Fußspitze 2T. Vorzugsweise weist die Bodenkontaktschicht die Außenseite 3 auf.

Die Bodenkontaktschicht ist vorzugsweise durch ein elastisches Material, insbesondere Polychloropren (Neopren), hergestellt.

Optional ist zwischen der Bodenkontaktschicht und dem Grundkörper eine Zwischenschicht vorgesehen, vorzugsweise wobei die Zwischenschicht aus einem Softshell-Material bzw. Polyester, Polyamid und/oder Elastan hergestellt ist.

Der Fußüberzieher 2, insbesondere der Grundkörper, ist vorzugsweise aus Naturfaser, beispielsweise Baumwolle oder Bambus, und/oder aus Kunstfaser, beispielsweise Polyester, Elastan oder dergleichen, hergestellt.

Vorzugsweise weist der Fußüberzieher 2, insbesondere der Grundkörper, die Bodenkontaktschicht und/oder die Zwischenschicht, eine (elastische) Dehnung (Stretch) von mindestens 60 % oder 70 %, insbesondere zumindest im Wesentlichen 80 %, auf.

Das Kit 1 weist darüber hinaus wenigstens ein Stützelement 4 auf, wie Fig. 1 zeigt. Das Stützelement 4 ist vorzugsweise als vom Fußüberzieher 2 separate Komponente ausgebildet.

Das Stützelement 4 ist insbesondere lösbar an dem Fußüberzieher 2, insbesondere der Bodenkontaktschicht, befestigbar oder lösbar mit dem Fußüberzieher 2, insbesondere der Bodenkontaktschicht, verbindbar. Im Tragezustand ist das Stützelement 4 insbesondere im Bereich der Fußsohle der tragenden Person bzw. des Fußüberziehers 2 angeordnet. Vorzugsweise ist das Stützelement 4 an der Außenseite 3 oder im Bereich der Außenseite 3 befestigbar bzw. mit dieser verbindbar.

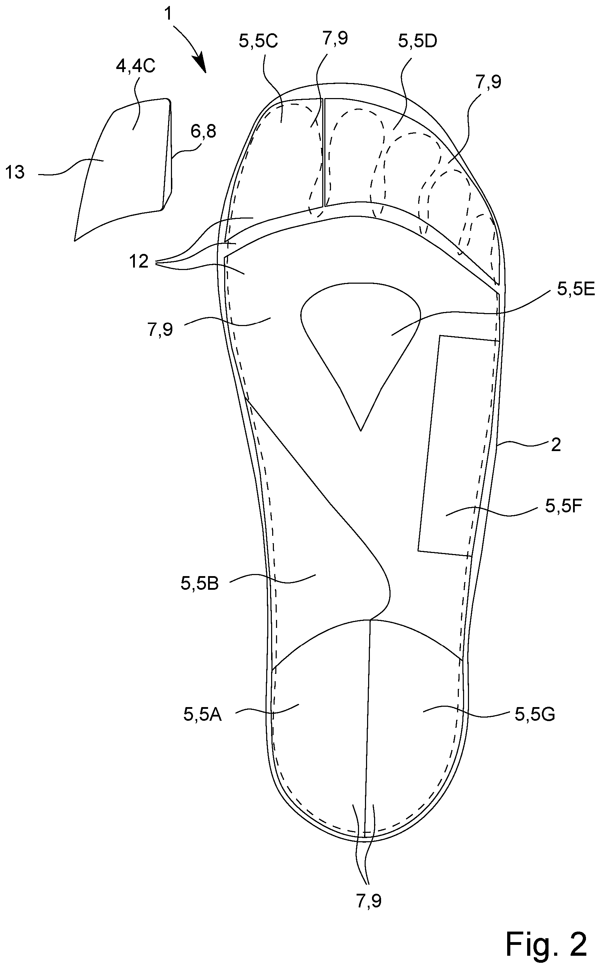

In Fig. 2 ist der Fußüberzieher 2 von unten mit dem separaten Stützelement 4 gezeigt. Das Stützelement 4 ist vorzugsweise an wenigstens zwei verschiedenen vorgegebenen Bereichen 5 des Fußüberziehers 2 lösbar befestigbar. Je nachdem, an welchem Bereich 5 das Stützelement 4 mit dem Fußüberzieher 2 verbunden ist, können unterschiedliche Fehlstellungen und/oder Fehlbelastungen des Bewegungsapparats ausgeglichen werden.

So kann die das Kit 1 nutzende Person bei einer ersten Fehlstellung und/oder Fehlbelastung des Bewegungsapparats das Stützelement 4 an einem ersten Bereich 5A befestigen. Beim Tragen des Kits 1 bzw. des mit dem Stützelement 4 verbundenen Fußüberziehers 2 kann die erste Fehlstellung und/oder Fehlbelastung ausgeglichen werden, wodurch mögliche Beschwerden, wie beispielsweise Schmerzen, der Person gelindert werden können.

Weist die Person eine zweite Fehlstellung und/oder Fehlbelastung des Bewegungsapparats auf, kann die Person das Stützelement 4 an dem zweiten Bereich 5B befestigen, um die zweite Fehlstellung und/oder Fehlbelastung des Bewegungsapparats auszugleichen.

Das Stützelement 4 ist vorzugsweise elastisch verformbar. Auf diese Weise kann sich das Stützelement 4 an die Belastungen durch die tragende Person anpassen und wird nicht als störend empfunden. Insbesondere kann sich das Stützelement 4 an die Form des Fußes in dem den Stützelement 4 zugeordneten Bereich 5 anpassen. Insgesamt kann auf diese Weise der Komfort für die tragende Person verbessert werden.

Das Stützelement 4 ist bevorzugt aus vorzugsweise elastisch verformbaren Kunststoff hergestellt. Das Stützelement 4 kann aus einem Kunststoff oder aus mehreren Kunststoffen hergestellt sein. So ist es beispielsweise möglich, das Stützelement 4 aus einem geschäumten, thermoplastischen und/oder faserverstärkten Kunststoff und/oder Silikon herzustellen. Insbesondere kann das Stützelement 4 mittels eines 3D-Druckverfahrens hergestellt sein. Das Kunststoffmaterial kann auch einen zumindest abschnittsweisen Überzug, beispielsweise aus einem Textil und/oder Kunststoff aufweisen.

Das Stützelement 4 kann auch aus Carbon hergestellt sein oder Carbon aufweisen. Es ist auch denkbar, dass das Stützelement 4 Metall aufweist, beispielsweise als das Stützelement 4 verstärkendes Einlageelement.

Das Stützelement 4 kann alternativ oder zusätzlich aus einem anderen Werkstoff, wie beispielsweise Kork und/oder Leder, hergestellt sein oder einen anderen Werkstoff, wie Kork und/oder Leder, aufweisen.

Das Stützelement 4 kann eine konstante oder variable Höhe aufweisen. Unter dem Begriff "Höhe" ist vorliegend die Erstreckung des Stützelements 4 im Tragezustand entlang der Richtung der Schwerkraft zu verstehen.

Das Stützelement 4 kann eine Höhe von mehr als 1 mm, vorzugsweise von mehr als 2 mm, weiter vorzugsweise von mehr als 3 mm, und/oder von weniger als 40 mm, vorzugsweise von weniger als 30 mm, weiter vorzugsweise von weniger als 20 mm, aufweisen.

Das Stützelement 4 kann eine dem Fußüberzieher 2 und/oder dem Fuß des Nutzers zugeordnete und/oder zugewandte Anlagefläche 6 und der Fußüberzieher 2 eine dem Stützelement 4 zugeordnete und/oder zugewandte Anlagegegenfläche 7 aufweisen, wie Fig. 2 bis Fig. 5 zeigen. Besonders bevorzugt sind die Anlagefläche 6 und die Anlagegegenfläche 7 zumindest im Wesentlichen identisch ausgebildet. Auf diese Weise kann eine besonders gleichmäßige und stabile Verbindung zwischen dem Stützelement 4 und dem Fußüberzieher 2 realisiert werden.

Es ist auch möglich, dass die Anlagefläche 6 und die Anlagegegenfläche 7 unterschiedlich ausgebildet sind.

Besonders bevorzugt entspricht die Größe und/oder Form des vorgegebenen Bereichs 5 der Größe und/oder Form der Anlagefläche 6 und/oder der Anlagegegenfläche 7. Die Anlagefläche 6 erstreckt sich somit vorzugsweise über den gesamten entsprechenden vorgegebenen Bereich 5.

**In** der in Fig. 2 gezeigten Ausführungsform ist die Verbindung zwischen dem Fußüberzieher 2 und dem Stützelement 4 mittels einer Klettverschlussverbindung realisiert, wodurch eine leichte Herstellung und Lösung der Verbindung ermöglicht wird.

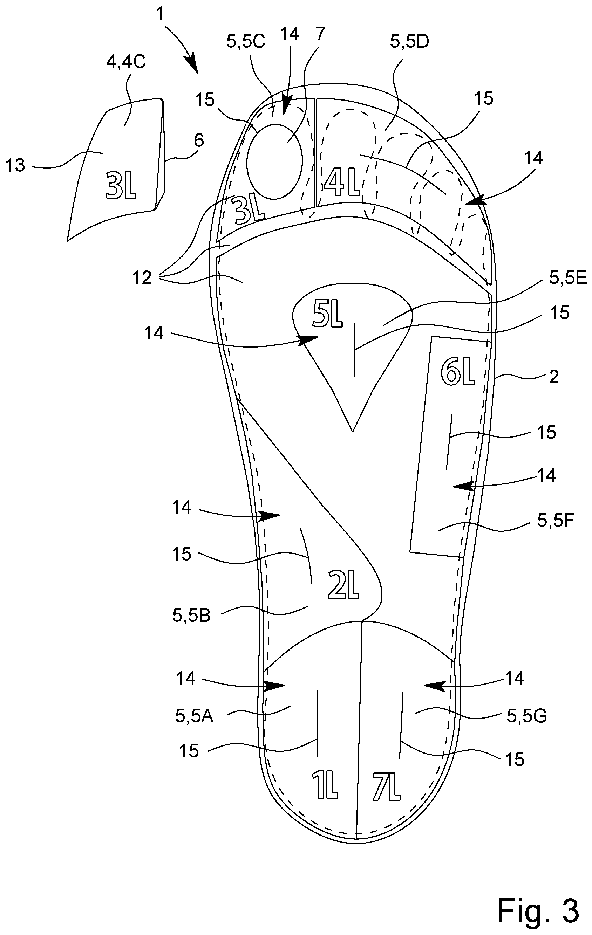

Die Anlagefläche 6 kann ein Klettverschlusselement 8 und/oder die Anlagegegenfläche 7 ein Klettverschlussgegenelement 9 aufweisen oder daraus gebildet sein, wie Fig. 2 des Weiteren zeigt. Das Stützelement 4 kann dann in besonders einfacher und wieder lösbarer Weise mittels der Klettverschlussverbindung mit dem Bereich 5 des Fußüberziehers 2 verbunden werden. In Fig. 3 ist der Fußüberzieher 2 mit dem verbundenen Stützelement 4 gezeigt.

In Fig. 3 bis Fig. 5 ist das Kit 1 gemäß einer weiteren Ausführungsform gezeigt. In der in Fig. 3 bis Fig. 5 gezeigten und insoweit bevorzugten Ausführungsform ist vorgesehen, dass jeder vorgegebene Bereich 5 jeweils eine Aufnahmetasche 14 zur Aufnahme eines Stützelements 4 aufweist. Alternativ kann jeder vorgegebene Bereich 5 durch eine Aufnahmetasche 14 zur Aufnahme eines Stützelements 4 gebildet sein.

Die Aufnahmetasche 14 ist vorzugsweise in der Bodenkontaktschicht vorgesehen.

Vorzugsweise sind die Bodenkontaktschicht und die Zwischenschicht bzw. der Grundkörper derart vernäht, dass sich die Aufnahmetasche 14 bildet bzw. die Aufnahmetasche 14 begrenzt wird. Insbesondere ist die Aufnahmetasche 14 durch die Bodenkontaktschicht nach außen und durch die Zwischenschicht bzw. den Grundkörper nach innen begrenzt.

Das Stützelement 4 ist vorzugsweise zur Befestigung an dem Fußüberzieher 2 in die Aufnahmetasche 14 einführbar. Insbesondere kann das Einführen des Stützelements 4 in die Aufnahmetasche 14 unmittelbar ursächlich für die Fixierung des Stützelements 4 in der Aufnahmetasche 14 und/oder an dem Fußüberzieher 2 sein.

Vorzugsweise entspricht die Form der Aufnahmetasche 14 bzw. des taschenförmigen Aufnahmeelements zumindest im Wesentlichen dem zugeordneten Stützelement 4, wodurch eine besonders stabile Fixierung erreicht werden kann.

Insbesondere kann die Form der Aufnahmetasche 14 bzw. des taschenförmigen Aufnahmeelements zumindest im Wesentlichen dem vorgegebenen Bereich 5 entsprechen.

Die Aufnahmetasche 14 bzw. die Bodenkontaktschicht kann insbesondere auf der Unterseite bzw. der dem Fuß abgewandten Seite eine Öffnung 15 aufweisen, wie Fig. 3 zeigt.

Vorzugsweise ist die Öffnung 15 schlitzartig ausgebildet und/oder zumindest im Wesentlichen mittig in der Aufnahmetasche 14 angeordnet.

Die Öffnung 15 ist vorzugsweise kleiner als das Stützelement 4.

Die Aufnahmetasche 14 bzw. Öffnung 15 kann derart dehnbar sein, dass die Größe der Öffnung 15 veränderbar ist. Insbesondere kann die Öffnung 15 von einem - zumindest im Wesentlichen ovalförmigen - Öffnungszustand, in dem das (größere) Stützelement 4 in die Aufnahmetasche 14 einführbar ist, in einen - zumindest im Wesentlichen schlitzartigen - Schließzustand überführbar sein, in dem das Stützelement 4 in der Aufnahmetasche 14 fixiert ist. Die reversible Dehnbarkeit der Aufnahmetasche 14 ist dann vorzugsweise ursächlich für die Fixierung des Stützelements 4.

Die Aufnahmetasche 14 kann insbesondere derart dehnbar sein, dass eine Verkleinerung der Öffnung 15 eine Fixierung des in der Aufnahmetasche 14 befindlichen Stützelements 4 bewirkt. Die Verkleinerung der Öffnung 15 ist dann ursächlich für die Fixierung des Stützelements 4.

Mit anderen Worten ist die Öffnung 15 derart vergrößerbar, dass das Stützelement 4 in die Aufnahmetasche 14 eingeführt werden kann.

Zur Entnahme des Stützelements 4 ist es dann lediglich notwendig, die Aufnahmetasche 14 bzw. die Öffnung 15 derart zu dehnen, dass die Öffnung 15 in den Öffnungszustand übergeht, und das Stützelement 4 aus der Aufnahmetasche 14 zu entnehmen. Hierzu wird die Öffnung 15 gegenüber dem Schließzustand vergrößert.

In Fig. 3 ist die Öffnung 15 des Bereichs 5C im Öffnungszustand gezeigt. Die Öffnungen 15 der anderen Bereiche 5A, 5B, 5D, 5E, 5F, 5G sind jeweils im Schließzustand gezeigt.

Wie bereits erläutert, ist die Öffnung 15 vorzugsweise zumindest im Wesentlichen mittig, also insbesondere nicht randseitig, im Bereich 5 bzw. der Aufnahmetasche 14 angeordnet. Auf diese Weise wird das Stützelement 4 sicher in Position gehalten. Bei einer randseitigen Anordnung besteht nämlich die Gefahr, dass das Stützelement 4 durch die Bewegung des Fußes aus der Aufnahmetasche 14 gedrückt wird.

Die Längserstreckung der schlitzartigen Öffnungen 15 kann parallel oder quer zur Längserstreckung des Fußüberziehers 2 bzw. Fußes verlaufen. In der dargestellten Ausführungsform verlaufen die Längserstreckung einer Öffnung 15, nämlich der Öffnung 15 im Bereich 5D, quer und die Längserstreckungen der übrigen Öffnungen 15 parallel zur Längserstreckung des Fußüberziehers 2 bzw. Fußes.

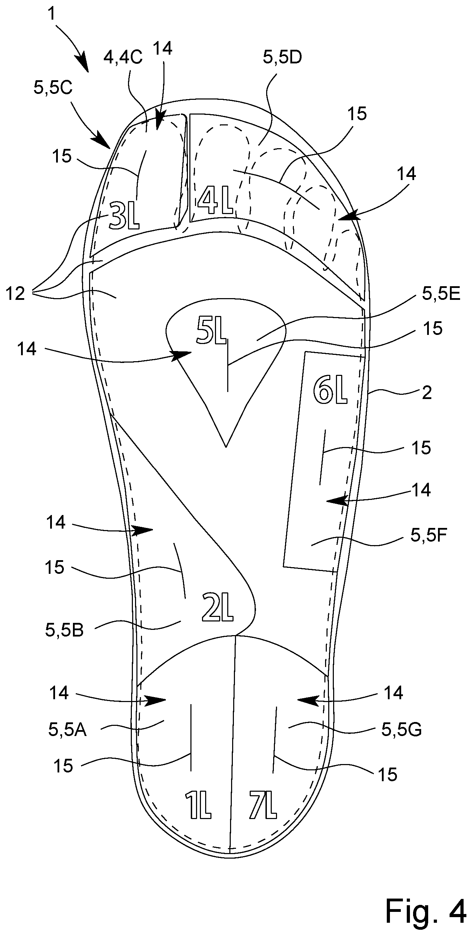

In Fig. 4 ist das Stützelement 4C in der dem Bereich 5C zugeordneten Aufnahmetasche 14 angeordnet und fixiert. Die Öffnungen 15 der Aufnahmetaschen 14 befinden sich jeweils im Schließzustand.

Wie Fig. 2 bis Fig. 5 zeigen weist der Fußüberzieher 2 vorzugsweise wenigstens drei, in den Figuren genau sieben, verschiedene vorgegebene Bereiche 5 auf.

Wie Fig. 2 bis Fig. 5 des Weiteren zeigen, sind die Bereiche 5 auf dem Fußüberzieher 2 insbesondere derart ausgebildet, dass die unterschiedlichen Bereiche 5 voneinander unterscheidbar sind. Die unterschiedlichen Bereiche 5 sind vorzugsweise optisch und/oder haptisch voneinander unterscheidbar.

Besonders bevorzugt sind die vorgegebenen Bereiche 5 bzw. Aufnahmetaschen 14 auf dem Fußüberzieher 2, insbesondere farblich und/oder schriftlich bzw. durch Zeichen, markiert. Auf diese Weise können die verschiedenen Bereiche 5 bzw. Aufnahmetaschen 14 in besonders einfacher Weise optisch voneinander unterschieden werden.

Die verschiedenen Bereiche 5 bzw. Aufnahmetaschen 14 können beispielsweise unterschiedliche Farben aufweisen, unterschiedlich beschriftet sein und/oder jeweils eine unterschiedliche Umrandung, beispielsweise durch eine Linie bzw. farbliche Naht, aufweisen. Auf diese Weise können die verschiedenen Bereiche 5 bzw. Aufnahmetaschen 14 durch die nutzende Person in besonders einfacher Weise gefunden und voneinander unterschieden werden.

In der in den Figuren gezeigten Ausführungsform weisen die vorgegeben Bereiche 5 bzw. Aufnahmetaschen 14 vorzugsweise eine linienförmige Umrandung insbesondere durch eine farbliche Naht auf. Die linienförmige Umrandung kann insbesondere eine andere Farbe als der Fußüberzieher 2 im Übrigen aufweisen.

Zusätzlich ist es möglich, dass die Bereiche 5 bzw. Aufnahmetaschen 14 des linken Fußüberziehers 2 unterschiedlich zu den Bereichen 5 bzw. Aufnahmetaschen 14 des rechten Fußüberziehers 2, insbesondere farblich und/oder schriftlich, markiert sind. Beispielsweise können die Bereiche 5 bzw. Aufnahmetaschen 14 des linken Fußüberziehers 2 eine andere farbliche Umrandung bzw. Naht aufweisen als die Bereiche 5 bzw. Aufnahmetaschen 14 des rechten Fußüberziehers 2.

Wie Fig. 2 bis Fig. 5 des Weiteren zeigen, sind die verschiedenen Bereiche 5 bzw. Aufnahmetaschen 14 vorzugsweise unterschiedlich ausgebildet. Unter dem Begriff "unterschiedlich ausgebildet" ist vorliegend zu verstehen, dass die verschiedenen Bereiche 5 unterschiedliche Formen und/oder Größen aufweisen.

Das Kit 1 weist vorzugsweise wenigstens zwei Stützelemente 4 auf. Im Tragezustand ist es möglich, dass lediglich ein Stützelement 4 an einem vorgegeben Bereich 5 befestigt ist. Alternativ ist es auch möglich, dass mehrere Stützelemente 4 an verschiedenen vorgegebenen Bereichen 5 des Fußüberziehers 4 lösbar befestigt sind.

Durch den Einsatz mehrerer Stützelemente 4 können mehr als eine Fehlstellung und/oder Fehlbelastung des Bewegungsapparats ausgeglichen werden. Auch ist es durch den Einsatz mehrerer Stützelemente 4 möglich, eine Fehlstellung und/oder Fehlbelastung des Bewegungsapparats besser ausgleichen zu können als bei der Verwendung nur eines Stützelements 4.

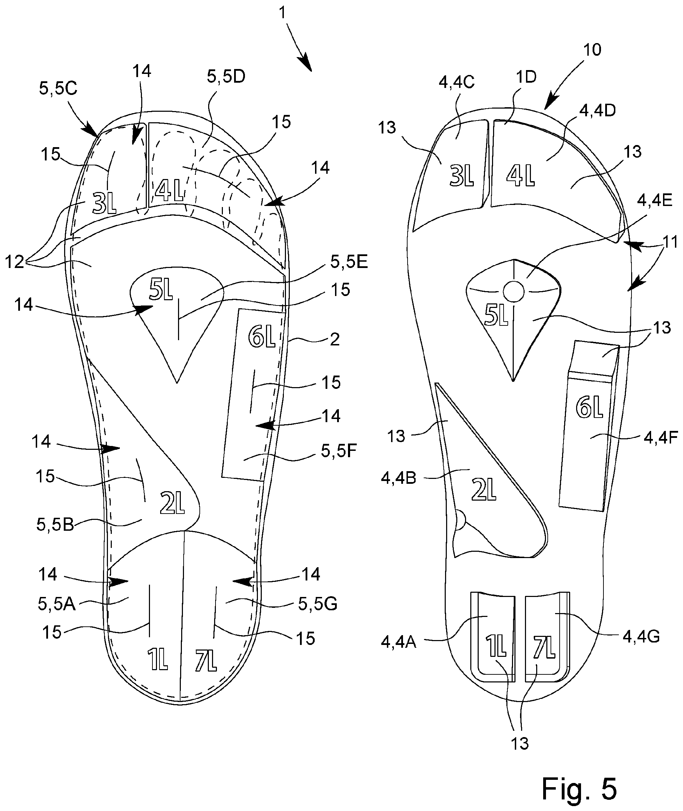

In Fig. 5 ist der Fußüberzieher 2 und die Stützelemente 4 in einer Ansicht von unten gezeigt. Wie sich Fig. 4 entnehmen lässt, weist das Kit 1 vorzugsweise genau sieben Stützelemente 4 und genau sieben Bereiche 5 bzw. Aufnahmetaschen 14 auf dem Fußüberzieher 2 auf.

Wie Fig. 5 weiterhin zeigt, sind die Stützelemente 4 vorzugsweise jeweils unterschiedlich ausgebildet. Die Stützelemente 4 können, insbesondere dreidimensional, unterschiedliche Größen, Konturen und/oder Formen aufweisen.

Wie Fig. 5 zeigt, ist vorzugsweise für jeden vorgegebenen Bereich 5 bzw. jede Aufnahmetasche 14 ein speziell ausgebildetes Stützelement 4 vorgesehen. Alternativ oder zusätzlich kann jedem vorgegebenen Bereich 5 bzw. jeder Aufnahmetasche 14 ein speziell ausgebildetes Stützelement 4 zugeordnet sein.

In Fig. 5 sind die sieben Bereich 5A bis 5G mit den zugehörigen Stützelementen 4A bis 4G gezeigt.

Wie Fig. 5 des Weiteren zeigt, weist der Fußüberzieher 2 vorzugsweise zwei Bereiche 5C, 5D auf, die den Zehen der Person zugeordnet sind. Ein Bereich 5C kann dabei dem großen Zeh bzw. Hallux zugeordnet sein. Alternativ oder zusätzlich kann ein Bereich 5D den Digitus II bis V zugeordnet sein.

Der dem großen Zeh bzw. Hallux zugeordnete Bereich 5C kann quer zur distalen Erstreckung eine größere Breite als der den Digitus II bis V zugeordnete Bereich 5D aufweisen. Wenigstens ein den Zehen zugeordneter Bereich 5C, 5D kann zumindest im Wesentlichen bogen- und/oder sichelförmig ausgebildet sein.

Wie Fig. 5 zeigt, ist können den beiden Bereichen 5C, 5D jeweils ein entsprechendes Stützelement 4C, 4D zugeordnet sein.

Die beiden Stützelemente 4C, 4D können den Zehen der Person zugeordnet sein, wobei das eine Stützelement 4C dem großen Zeh bzw. Hallux und das andere Stützelement 4D den Digitus II bis V zugeordnet sein kann.

Das dem großen Zeh bzw. Hallux zugeordnete Stützelement 4C kann quer zur distalen Erstreckung eine größere Breite als das den Digitus II bis V zugeordnete Stützelement 4D aufweisen.

Das dem großen Zeh bzw. Hallux zugeordnete Stützelement 4C und/oder das den Digitus II bis V zugeordnete Stützelement 4D kann zumindest im Wesentlichen bogen- und/oder sichelförmig ausgebildet sein.

Wie Fig. 5 des Weiteren zeigt, können drei Bereiche 5B, 5E, 5F des Fußüberziehers 2 dem Mittelfuß oder einem Bereich des Mittelfußes der Person zugeordnet sein. Die drei Bereiche 5B, 5E, 5F können sich ausgehend von den Zehen zur Ferse erstrecken, insbesondere vom Übergang zwischen den Zehen und dem Mittelfuß bis zum Übergang vom Mittelfuß zur Ferse.

Der eine Bereich 5B kann der Innenseite, der eine Bereich 5F der Außenseite und/oder der eine Bereich 5E dem Innenbereich des Mittelfußes zugeordnet sein.

Bezogen auf die Richtung quer zur distalen Erstreckung kann der der Innenseite zugeordnete Bereich 5B breiter als der der Außenseite zugeordnete Bereich 5F ausgebildet sein.

Das Kit 1 weist vorzugsweise drei Stützelemente 4B, 4E, 4F auf, die dem Mittelfuß der Person zugeordnet sind. Die drei Stützelemente 4B, 4E, 4F können sich ausgehend von den Zehen zur Ferse erstrecken, insbesondere vom Übergang zwischen den Zehen und dem Mittelfuß bis zum Übergang vom Mittelfuß zur Ferse.

Das eine Stützelement 4B kann der Innenseite, das eine Stützelement 4F der Außenseite und/oder das eine Stützelement 4E dem Innenbereich des Mittelfußes zugeordnet sein.

Bezogen auf die Richtung quer zur distalen Erstreckung kann das der Innenseite zugeordnete Stützelement 4B breiter als das der Außenseite zugeordnete Stützelement 4F ausgebildet sein.

Wie aus Fig. 5 hervorgeht, können zwei Bereiche 5A, 5G der Ferse der Person zugeordnet sein. Der eine Bereich 5 kann der Innenseite bzw. der inneren Hälfte der Ferse und der ein Bereich 5G der Außenseite bzw. der äußeren Hälfte der Ferse, zugeordnet sein.

Die beiden Bereiche 5A, 5G können vorzugsweise bezogen auf die Richtung quer zur distalen Richtung eine zumindest im Wesentlichen gleiche Breite aufweisen, wobei auch unterschiedliche Breiten möglich sind.

Das Kit 1 weist vorzugsweise zwei den Bereichen 5A, 5G zugeordnete Stützelemente 4A, 4G auf. Die Stützelemente 4A, 4G sind vorzugsweise der Ferse der Person zugeordnet. Das eine Stützelement 4A kann der inneren Hälfte der Ferse und das andere Stützelement 4G der äußeren Hälfte der Ferse zugeordnet sein.

Die dem Mittelfuß zugeordneten Bereiche 5B, 5E, 5F können in distaler Richtung eine größere Erstreckung aufweisen als die den Zehen zugeordneten Bereiche 5C, 5D und/oder die der Ferse zugeordneten Bereiche 5A, 5G.

Die dem Mittelfuß zugeordneten Stützelemente 4B, 4E, 4F können in distaler Richtung eine größere Erstreckung aufweisen als die den Zehen zugeordneten Stützelemente 4C, 4D und/oder die der Ferse zugeordneten Stützelemente 4A, 4G, wie Fig. 5 zeigt.

Die der Ferse zugeordneten Bereiche 5A, 5G des Fußüberziehers 2 eine größere Erstreckung in distaler Richtung aufweisen als die den Zehen zugeordneten Bereiche 5C, 5D. Wie Fig. 5 des Weiteren zeigt, können die der Ferse zugeordneten Stützelemente 4A, 4G eine größere Erstreckung in distaler Richtung aufweisen als die den Zehen zugeordneten Stützelemente 4C, 4D.

Der dem Innenbereich des Mittelfußes zugeordnete Bereich 5E und/oder das dem Innenbereich des Mittelfußes zugeordnete Stützelement 4E kann zumindest im Wesentlichen V-förmig, tropfenförmig und/oder drachendreieckförmig ausgebildet sein.

Weist die tragende Person beispielsweise einen Knickfuß auf, kann das Tragen des Fußüberziehers 2, der mit den Stützelementen 4A und 4B an den Bereichen 5A und 5B verbunden ist, die entsprechende Fehlstellung und/oder Fehlbelastung des Bewegungsapparats der Person ausgleichen.

Bei einem Hohlfuß können beispielsweise die Stützelemente 4B, 4E und 4F an den Bereichen 5B, 5E und 5F zum Ausgleich der Fehlstellung und/oder Fehlbelastung des Bewegungsapparats verwendet werden. Bei Fehlstellungen und/oder Fehlbelastungen des Bewegungsapparats ausgelöst durch eine Fehlstellung und/oder Krankheit im Bereich des Vorfußes, kann die gleiche Anordnung verwendet werden.

Um die Fehlstellung und/oder Fehlbelastung bei einem Spreizfuß auszugleichen, kann das Stützelement 4E am Bereich 5E befestigt werden.

Zum Ausgleich der Fehlstellung und/oder Fehlbelastung des Bewegungsapparats bei Krallenzehen kann der Fußüberzieher 2 am Bereich 5D mit dem Stützelement 4D verbunden und von der Person getragen werden.

Weist eine Person Wadenkrämpfe auf, kann das Tragen des Fußüberziehers 2 mit den Stützelementen 4C, 4D in den Bereichen 5C, 5D die Fehlbelastung und/oder Fehlstellung des Bewegungsapparats ausgleichen.

Bei einem Fersensporn können die Stützelemente 4A, 4G an den Bereichen 5A, 5G verwendet werden.

Bei Knieschmerzen an den Innenseiten und/oder bei O-Beinen können die Stützelemente 4G, 4F an den Bereichen 5G, 5F befestigt und der Fußüberzieher 2 entsprechend getragen werden.

Bei Knieschmerzen an der Außenseite und/oder bei X-Beinen können die Stützelemente 4A, 4B an den Bereichen 5A, 5B befestigt und der Fußüberzieher 2 entsprechend getragen werden.

Die Stützelemente 4 können unterschiedlich markiert sein, insbesondere um die Stützelementen 4 dem linken oder rechten Fußüberzieher 2 und/oder den Bereichen 5 bzw. Aufnahmetaschen 14 des (linken oder rechten) Fußüberziehers 2 eindeutig zuzuordnen, beispielsweise durch unterschiedliche Farben und/oder jeweils eine Beschriftung. Ganz besonders bevorzugt korrespondiert die Markierung der Stützelemente 4 zu der Markierung der Bereiche 5 bzw. Aufnahmetaschen 14 auf dem Fußüberzieher 2.

Insbesondere können die Stützelemente 4 und Bereiche 5 bzw. Aufnahmetaschen 14 mit zueinander korrespondierenden Nummern markiert sein. Beispielsweise kann der Bereich 5A und das zugeordnete Stützelement 4A mit einer "1", der Bereich 5B und das zugeordnete Stützelement 4B mit einer "2", der Bereich 5C und das zugeordnete Stützelement 4C mit einer "3", der Bereich 5D und das zugeordnete Stützelement 4D mit einer "4", der Bereich 5E und das zugeordnete Stützelement 4E mit einer "5", der Bereich 5F und das zugeordnete Stützelement 4F mit einer "6" und der Bereich 5G und das zugeordnete Stützelement 4G mit einer "7" markiert sein. Zusätzlich können weitere Markierungen, wie "R" und "L" vorgesehen sein, um die Stützelemente 4 eindeutig dem linken oder rechten Fußüberzieher 2 zuzuordnen.

Zur Aufbewahrung nicht verwendeter Stützelemente 4 kann das Kit 1 ein Aufnahmeelement 10 aufweisen. Das Aufnahmeelement 10 kann insbesondere nach Art einer Sohle, insbesondere Einlegesohle, ausgebildet sein, wie Fig. 5 zeigt. Das Aufnahmeelement 10 kann mit den Stützelementen 4, insbesondere lösbar, verbindbar sein. Die nicht verwendeten Stützelemente 4 können dann mit dem Aufnahmeelement 10 verbunden bleiben oder mit dem Aufnahmeelement 10 verbunden werden. Insbesondere kann jedem Stützelement 4 ein Aufnahmeabschnitt 11 des Aufnahmeelements 10 zugeordnet sein.

Die Aufnahmeabschnitte 11 können derart ausgebildet sein, dass sie voneinander unterscheidbar sind, insbesondere optisch. Die Aufnahmeabschnitte 11 können hierfür farblich unterschiedlich markiert, beschriftet und/oder mittels einer Linie umrandet sein.

Es ist auch möglich, dass das Aufnahmeelement 10 als Aufbewahrungsbox, insbesondere mit einer wieder lösbar verschließbaren Öffnung, oder dergleichen ausgebildet ist.

Wie Fig. 5 zeigt, kann der Fußüberzieher 2, insbesondere die Bodenkontaktschicht, eine der Fußsohle abgewandte Bodenkontaktfläche 12 aufweisen. Der Fußüberzieher 2, insbesondere die Bodenkontaktschicht, weist insbesondere zumindest in einem Abschnitt der Bodenkontaktfläche 12 eine Anti-Rutsch-Schicht auf. Die Anti-Rutsch-Schicht ist vorzugsweise dazu ausgebildet, das Ausrutschen der tragenden Person auf einem glatten Untergrund zu verhindern. Hierfür kann die Anti-Rutsch-Schicht die Reibung zwischen der Anti-Rutsch-Schicht und dem Untergrund im Vergleich zum Fußüberzieher 2 im Übrigen erhöhen. Auf diese Weise kann der Tragekomfort und die Sicherheit für die tragende Person erhöht werden.

Alternativ oder zusätzlich kann das Stützelement 4 im Tragezustand eine der Fußsohle abgewandte Bodenfläche 13 aufweisen, wie Fig. 1 zeigt. Die Bodenfläche 13 kann zumindest abschnittsweise als Anti-Rutsch-Schicht ausgebildet sein oder eine Anti-Rutsch-Schicht aufweisen.

**Bezugszeichenliste:**

| | | | |
|---|---|---|---|
| 1 | Kit | 5A | Bereich |
| 2 | Fußüberzieher | 5B | Bereich |
| 2B | Bündchen | 5C | Bereich |
| 2C | Fußteil | 5D | Bereich |
| 2F | Ferse | 5E | Bereich |
| 2S | Schaft | 5F | Bereich |
| 2T | Fußspitze | 5G | Bereich |
| 3 | Außenseite | 6 | Verbindungsfläche |
| 4 | Stützelement | 7 | Verbindungsgegenfläche |
| 4A | Stützelement | 8 | Klettverschlusselement |
| 4B | Stützelement | 9 | Klettverschlussgegenelement |
| 4C | Stützelement | 10 | Aufnahmeelement |
| 4D | Stützelement | 11 | Aufnahmeabschnitt |
| 4E | Stützelement | 12 | Bodenkontaktfläche |
| 4F | Stützelement | 13 | Bodenfläche |
| 4G | Stützelement | 14 | Aufnahmetasche |
| 5 | Bereich | 15 | Öffnung |

## Patentansprüche

1. Kit zum Ausgleich einer Fehlstellung und/oder Fehlbelastung des Bewegungsapparats einer Person,
mit einem Fußüberzieher (2) für einen Fuß der Person,
wobei der Fußüberzieher (2) im Tragezustand wenigstens einen Abschnitt des Fußes bedeckt und/oder den Fuß zumindest abschnittsweise vollumfänglich umgibt bzw. umschließt, wobei der Fußüberzieher (2) nach Art einer Socke, einer Schlauchbandage und/oder einer Knöchelbandage ausgebildet ist,
und mit wenigstens einem Stützelement (4) zum Stützen und/oder Anheben eines Fußabschnitts,
wobei der Fußüberzieher (2) wenigstens drei unterschiedliche vorgegebene Bereiche (5) aufweist, wobei im Tragezustand ein Stützelement (4) an einem vorgegebenen Bereich (5) oder eine Mehrzahl an Stützelementen (4) an verschiedenen vorgegebenen Bereichen (5) lösbar befestigt ist,
wobei jeder vorgegebene Bereich (5) jeweils eine Aufnahmetasche (14) zur Aufnahme eines Stützelements (4) aufweist oder durch eine Aufnahmetasche (14) zur Aufnahme eines Stützelements (4) gebildet ist, wobei das Stützelement (4) zur Befestigung an dem Fußüberzieher (2) in die Aufnahmetasche (14) einführbar ist, und
wobei die Aufnahmetasche (14) eine Öffnung (15) aufweist und derart dehnbar ist, dass die Größe der Öffnung (15) derart veränderbar ist, dass die Öffnung (15) von einem Öffnungszustand, in dem das Stützelement (14) in die Aufnahmetasche (14) einführbar ist, in einen Schließzustand überführbar ist, in dem das Stützelement (4) in der Aufnahmetasche (14) fixiert ist.

2. Kit nach Anspruch 1, wobei der Fußüberzieher (2) wenigstens vier, weiter vorzugsweise wenigstens fünf, weiter vorzugsweise wenigstens sechs, weiter vorzugsweise genau sieben, unterschiedliche vorgegebene Bereiche (5) aufweist.

3. Kit nach Anspruch 1 oder 2, wobei die vorgegebenen Bereiche (5) auf dem Fußüberzieher (2) derart ausgebildet sind, dass die verschiedenen Bereiche (5) voneinander unterscheidbar sind.

4. Kit nach einem der voranstehenden Ansprüche, wobei die vorgegebenen Bereiche (5) auf dem Fußüberzieher (2) und/oder die Stützelemente (4), insbesondere farblich, markiert sind.

5. Kit nach einem der voranstehenden Ansprüche, wobei das Stützelement (4) zumindest teilweise elastisch verformbar ist und/oder aus einem Kunststoffmaterial, insbesondere Silikon, hergestellt ist.

6. Kit nach einem der voranstehenden Ansprüche, wobei das Kit (1) wenigstens zwei, vorzugsweise wenigstens drei, weiter vorzugsweise genau sieben, Stützelemente (4) aufweist, insbesondere wobei im Tragezustand ein oder mehrere Stützelemente (4) an verschiedenen vorgegebenen Bereichen (5) des Fußüberziehers (2) lösbar befestigt sind.

7. Kit nach einem der voranstehenden Ansprüche, wobei die Stützelemente (4) unterschiedlich ausgebildet sind, und/oder
wobei die Bereiche (5) unterschiedlich ausgebildet sind.

8. Kit nach einem der voranstehenden Ansprüche, wobei die Anzahl der vorgegebenen Bereiche (5) der Anzahl der Stützelemente (4) entspricht, wobei jedem vorgegebenen Bereich (5) genau ein Stützelement (4) und/oder jedem Stützelement (4) genau ein vorgegebener Bereich (5) zugeordnet ist.

9. Kit nach einem der voranstehenden Ansprüche, wobei die Öffnung (15) schlitzartig ausgebildet und/oder zumindest im Wesentlichen mittig in dem Bereich (5) angeordnet ist.

10. Kit nach einem der voranstehenden Ansprüche, wobei der Fußüberzieher (2) zumindest bereichsweise, insbesondere auf einer Unterseite, mehrlagig ausgebildet ist.

11. Kit nach Anspruch 10, wobei der Fußüberzieher (2) einen Grundkörper und eine Bodenkontaktschicht aufweist, wobei der Grundkörper und die Bodenkontaktsicht kraft- und/oder stoffschlüssig miteinander verbunden sind und/oder die Aufnahmetasche (14) bilden.

12. Kit nach einem der voranstehenden Ansprüche, wobei das Stützelement (4) eine dem Fußüberzieher (2) zugewandte Anlagefläche (6) und der Fußüberzieher (2) eine dem Stützelement (4) zugeordnete und zugewandte Anlagegegenfläche (7) aufweist, vorzugsweise wobei die Anlagefläche (6) und die Anlagegegenfläche (7) zumindest im Wesentlichen gleich ausgebildet sind und/oder die Größe und/oder Form des vorgegebenen Bereichs (5) der Anlagefläche (6) und/oder der Anlagegegenfläche (7) entspricht.

13. Kit nach einem der voranstehenden Ansprüche, wobei zwei Bereiche (5C, 5D) den Zehen der Person zugeordnet sind, insbesondere wobei ein Bereich (5C) dem großen Zeh bzw. Hallux und/oder ein Bereich (5D) den Digitus II bis V zugeordnet ist, und/oder
wobei drei Bereiche (5B, 5E, 5F) dem Mittelfuß der Person zugeordnet sind und/oder sich ausgehend von den Zehen zur Ferse erstrecken, insbesondere wobei ein Bereich (5B) der Innenseite, ein Bereich (5F) der Außenseite und/oder ein Bereich (5E) dem Innenbereich des Mittelfußes zugeordnet ist, und/oder
wobei zwei Bereiche (5A, 5G) der Ferse der Person zugeordnet sind, insbesondere wobei ein Bereich (5A) der inneren Hälfte der Ferse und ein Bereich (5G) der äußeren Hälfte der Ferse zugeordnet ist.

14. Kit nach Anspruch 13, wobei die dem Mittelfuß zugeordneten Bereiche (5B, 5E, 5F) in distaler Richtung eine größere Erstreckung aufweisen als die den Zehen zugeordneten Bereiche (5C, 5D) und/oder die der Ferse zugeordneten Bereiche (5A, 5G), und/oder
wobei die der Ferse zugeordneten Bereiche (5A, 5G) eine größere Erstreckung in distaler Richtung aufweisen als die den Zehen zugeordneten Bereiche (5C, 5D), und/oder
wobei der dem Innenbereich des Mittelfußes zugeordnete Bereich (5E) zumindest im Wesentlichen V-förmig und/oder tropfenförmig und/oder drachenviereckförmig ausgebildet ist.

15. Kit nach einem der voranstehenden Ansprüche, wobei das Kit (1) ein vom Fußüberzieher (2) separates Aufnahmeelement (10) zur Aufnahme des Stützelements (4) aufweist, vorzugsweise wobei das Aufnahmeelement (10) nach Art einer Schuhsohle, insbesondere Schuheinlegesohle, ausgebildet ist.
